# EUROPEAN PATENT APPLICATION

(11) **EP 3 542 724 A1**
(43) Date of publication of application: **25.09.2019**
(21) Application number: 17872270.8
(22) Date of filing: 05.04.2017
(51) Int. Cl.: A61B 8/08, A61B 8/00

(54) **ULTRASONIC DIAGNOSTIC DEVICE AND OPERATION METHOD THEREOF**

(30) Priority: 18.11.2016 KR 20160154450
(71) Applicant: Samsung Medison Co., Ltd., Hongcheon-gun, Gangwon-do 25108 (KR)
(72) Inventor: KIM, Jun-seong, Hongcheon-gun Gangwon-do 25108 (KR)
(74) Representative: Hylarides, Paul Jacques
(86) International application number: PCT/KR2017/003722
(87) International publication number: WO 2018/092993

(57) **Abstract**

Provided is an ultrasound diagnosis apparatus including: a probe configured to receive echo signals from a fetus; a processor configured to acquire Doppler data based on the received echo signals, determine a heart rate of the fetus based on period information of the Doppler data, and modulate, based on the determined heart rate, a heartbeat Doppler sound of the fetus, which is acquired from the Doppler data, into a heartbeat sound that is similar to a sound of an actual heartbeat; and a speaker configured to output the heartbeat sound.

## Description

### Technical Field

The present disclosure relates to ultrasound diagnosis apparatuses and methods of operating the same.

### Background Art

Ultrasound diagnosis apparatuses transmit ultrasound signals generated by transducers of a probe to an object and receive echo signals reflected from the object, thereby obtaining at least one image of an internal part of the object.

In particular, ultrasound diagnosis apparatuses are used for medical purposes including observation of the interior of an object, detection of foreign substances, and diagnosis of damage to the object.

Such ultrasound diagnosis apparatuses provide high stability, display images in real time, and are safe due to the lack of radioactive exposure, compared to X-ray apparatuses. Therefore, ultrasound imaging apparatuses are widely used together with other image diagnosis apparatuses.

### Disclosure

### Technical Problem

Provided are ultrasound diagnosis apparatuses capable of providing a fetal heartbeat Doppler sound as well as a heartbeat sound that is similar to a sound of an actual heartbeat, thereby offering a high level of maternal empathy towards a fetus and various types of fetal heartbeat sounds.

Provided are non-transitory computer-readable recording media having recorded thereon a program for performing the method of operating the ultrasound diagnosis apparatus.

### Technical Solution

Additional aspects will be set forth in part in the description which follows and, in part, will be apparent from the description, or may be learned by practice of the presented embodiments.

According to an aspect of an embodiment, an ultrasound diagnosis apparatus includes: a probe configured to receive echo signals from a fetus; a processor configured to acquire Doppler data based on the received echo signals, determine a heart rate of the fetus based on period information of the Doppler data, and modulate, based on the determined heart rate, a heartbeat Doppler sound of the fetus, which is acquired from the Doppler data, into a heartbeat sound that is similar to a sound of an actual heartbeat; and a speaker configured to output the heartbeat sound.

The processor is further configured to acquire gestational age information of the fetus based on the Doppler data and modulate the heartbeat Doppler sound into the heartbeat sound according to the gestational age information and the heart rate of the fetus.

The gestational age information of the fetus includes at least one of a gestational age of the fetus, and head circumference, abdominal circumference, femur length, abdominal thickness, transverse trunk diameter and crown rump length, all of which correspond to the gestational age of the fetus.

The processor is further configured to compare the heart rate with a standard heart rate of a plurality of fetuses corresponding to gestational age information of the fetus, determine modulation information based on a result of the comparing of the heart rate with the standard heart rate and a standard heartbeat sound for the plurality of fetuses, and modulate the heartbeat Doppler sound into the heartbeat sound based on the modulation information.

The ultrasound diagnosis apparatus further includes a user interface configured to control an operation of the ultrasound diagnosis apparatus, wherein the user interface is further configured to receive an input for determining a type of the heartbeat sound, and wherein the processor is further configured to modulate the heartbeat Doppler sound into the heartbeat sound according to the type of the heartbeat sound.

The type of the heartbeat sound comprises at least one of a heartbeat sound heard through a stethoscope, a preset resounding heartbeat sound, and a preset majestic heartbeat sound.

The ultrasound diagnosis apparatus further includes a display, wherein the processor is further configured to control the speaker to output the heartbeat sound when an ultrasound image in which a focus is on a fetus' s heart is displayed on the display.

The ultrasound diagnosis apparatus further includes a communication module configured to transmit the heartbeat sound to an external terminal.

The ultrasound diagnosis apparatus further includes a display configured to display at least one of an ultrasound image of the fetus, a Doppler spectrum of the fetus, and heart rate information thereof.

The display is further configured to display a waveform of the heartbeat sound.

The display is further configured to display the heart rate of the fetus and the waveform of the heartbeat sound to be respectively distinguished from a standard heart rate of a plurality of fetuses corresponding to gestational age information of the fetus and a waveform of a standard heartbeat sound for the plurality of fetuses.

The processor is further configured to control the speaker to output prenatal music based on a state of the heart rate of the fetus.

According to an aspect of another embodiment, a method of operating an ultrasound diagnosis apparatus, the method includes: acquiring echo signals from a fetus and acquiring Doppler data based on the echo signals, determining a heart rate of the fetus based on period information of the Doppler data; modulating, based on the determined heart rate, a heartbeat Doppler sound of the fetus, which is acquired from the Doppler data, into a heartbeat sound that is similar to a sound of an actual heartbeat; and outputting the heartbeat sound.

The method further includes acquiring gestational age information of the fetus based on the Doppler data, wherein the modulating of the heartbeat Doppler sound into the heartbeat sound comprises modulating the heartbeat Doppler sound into the heartbeat sound according to the gestational age information and the heart rate of the fetus.

The modulating of the heartbeat Doppler sound into the heartbeat sound includes: comparing the heart rate with a standard heart rate of a plurality of fetuses corresponding to gestational age information of the fetus; determining modulation information based on a result of the comparing of the heart rate with the standard heart rate and a standard heartbeat sound for the plurality of fetuses; and modulating the heartbeat Doppler sound into the heartbeat sound based on the modulation information.

The method further includes receiving an input for determining a type of the heartbeat sound via a user interface configured to control an operation of the ultrasound diagnosis apparatus, wherein the modulating of the heartbeat Doppler sound into the heartbeat sound comprises modulating the heartbeat Doppler sound into the heartbeat sound according to the type of the heartbeat sound.

The outputting of the heartbeat sound comprises outputting the heartbeat sound when an ultrasound image in which a focus is on a fetus' s heart is displayed on a display of the ultrasound diagnosis apparatus.

The method further includes transmitting the heartbeat sound to an external terminal.

The method further includes displaying at least one of an ultrasound image of the fetus, a Doppler spectrum of the fetus, and heart rate information thereof on a display of the ultrasound diagnosis apparatus.

The method further includes displaying, on a display of the ultrasound diagnosis apparatus, the heart rate of the fetus and a waveform of the heartbeat sound to be respectively distinguished from a standard heart rate of a plurality of fetuses corresponding to gestational age information of the fetus and a waveform of a standard heartbeat sound for the plurality of fetuses.

According to an aspect of another embodiment, a non-transitory computer-readable recording medium has recorded thereon a method of operating an ultrasound diagnosis, which includes: acquiring echo signals from a fetus and acquiring Doppler data based on the echo signals; determining a heart rate of the fetus based on period information of the Doppler data; modulating, based on the determined heart rate, a heartbeat Doppler sound of the fetus acquired from the Doppler data into a heartbeat sound that is similar to a sound of an actual heartbeat; and outputting the resulting modulated heartbeat sound.

### Advantageous Effects

Provided are ultrasound diagnosis apparatuses capable of providing a fetal heartbeat Doppler sound as well as a heartbeat sound that is similar to a sound of an actual heartbeat, thereby offering a high level of maternal empathy towards a fetus and various types of fetal heartbeat sounds.

### Description of Drawings

These and/or other aspects will become apparent and more readily appreciated from the following description of the embodiments, taken in conjunction with the accompanying drawings in which:
FIG. 1 is a block diagram of a configuration of an ultrasound diagnosis apparatus according to an embodiment;
FIG. 2 is a block diagram of a configuration of a wireless probe according to an embodiment;
FIG. 3 is a block diagram of a configuration of an ultrasound diagnosis apparatus according to an embodiment;
FIG. 4 is a block diagram of a configuration of an ultrasound diagnosis apparatus according to another embodiment;
FIG. 5 is a flowchart of a method of operating an ultrasound diagnosis apparatus, according to an embodiment;
FIG. 6 is a detailed flowchart of an operation of generating a heartbeat sound in a method of operating an ultrasound diagnosis apparatus, according to an embodiment;
FIG. 7 is a table for explaining heartbeat sounds provided by an ultrasound diagnosis apparatus based on a gestational age and a heart rate of a fetus, according to an embodiment;
FIG. 8 is a flowchart of a method of operating an ultrasound diagnosis apparatus, according to another embodiment;
FIG. 9 illustrates a user interface provided in an ultrasound diagnosis apparatus, according to an embodiment;
FIG. 10 illustrates a screen of a user interface provided in an ultrasound diagnosis apparatus, according to an embodiment;
FIG. 11 is a flowchart of a method of operating an ultrasound diagnosis apparatus, according to another embodiment;
FIG. 12 illustrates a screen of a display on which an ultrasound diagnosis apparatus displays an ultrasound image, according to an embodiment;
FIG. 13 illustrates a screen of a display on which an ultrasound diagnosis apparatus displays an ultrasound image, according to another embodiment; and
FIG. 14 illustrates a screen of a display on which an ultrasound diagnosis apparatus displays data related to an ultrasound image, according to an embodiment.

### Best Mode

Provided is an ultrasound diagnosis apparatus including: a probe configured to receive echo signals from a fetus; a processor configured to acquire Doppler data based on the received echo signals, determine a heart rate of the fetus based on period information of the Doppler data, and modulate, based on the determined heart rate, a heartbeat Doppler sound of the fetus, which is acquired from the Doppler data, into a heartbeat sound that is similar to a sound of an actual heartbeat; and a speaker configured to output the heartbeat sound.

### Mode for Invention

The terms used in this specification are those general terms currently widely used in the art in consideration of functions regarding the inventive concept, but the terms may vary according to the intention of those of ordinary skill in the art, precedents, or new technology in the art. Also, some terms may be arbitrarily selected by the applicant, and in this case, the meaning of the selected terms will be described in detail in the detailed description of the present specification. Thus, the terms used in the specification should be understood not as simple names but based on the meaning of the terms and the overall description of the invention.

When a part "includes" or "comprises" an element, unless there is a particular description contrary thereto, the part can further include other elements, not excluding the other elements. Also, the term "unit" in the specification means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with a smaller number of components and "units", or may be divided into additional components and "units".

While such terms as "first," "second," etc., may be used to describe various components, such components are not limited thereto. These terms are used only to distinguish one component from another. For example, a first component may be referred to as a second component without departing from the scope of the inventive concept, and similarly, a second component may be referred to as a first component. As used herein, the term "and/or" includes any and all combinations of one or more of the associated listed items. Expressions such as "at least one of," when preceding a list of elements, modify the entire list of elements and do not modify the individual elements of the list.

In the present specification, an "image" may refer to multi-dimensional data composed of discrete image elements (e.g., pixels in a two-dimensional (2D) image and voxels in a three-dimensional (3D) image).

Throughout the specification, an "ultrasound image" refers to an image of an object, which is obtained using ultrasound waves. An ultrasound image may denote an image obtained by irradiating an ultrasound signal generated from a transducer of a probe to an object, and receiving information of an echo signal reflected by the object. Also, the ultrasound image may be variously implemented, and for example, the ultrasound image may be at least one of an amplitude (A) mode image, a brightness (B) mode image, a color (C) mode image, and a Doppler (D) mode image. Also, the ultrasound image may be a two-dimensional (2D) image or a three-dimensional (3D) image.

Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism.

Throughout the specification, a "user" may be, but is not limited to, a medical expert, for example, a medical doctor, a nurse, a medical laboratory technologist, or a medical imaging expert, or a technician who repairs medical apparatuses.

Reference will now be made in detail to embodiments, examples of which are illustrated in the accompanying drawings. In this regard, the present embodiments may have different forms and should not be construed as being limited to the descriptions set forth herein.

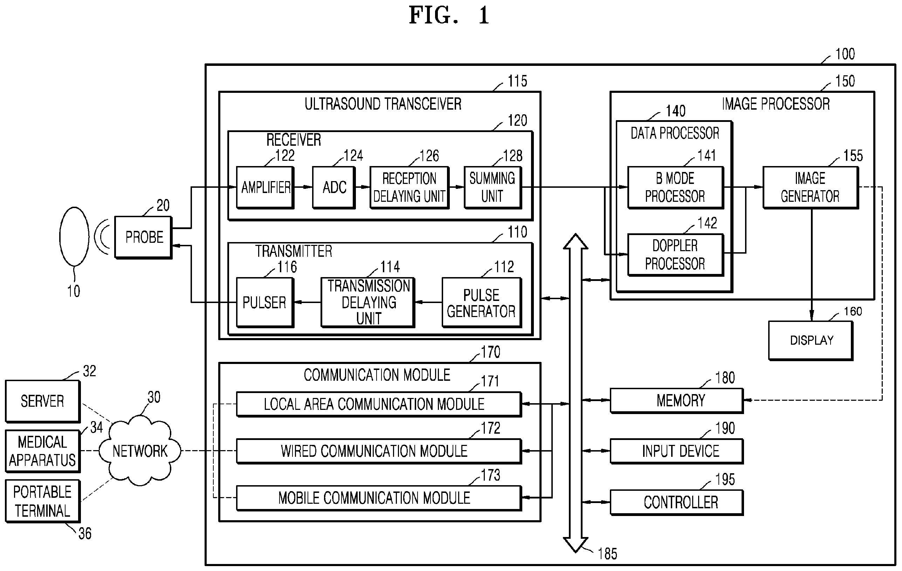

FIG. 1 is a block diagram showing a configuration of an ultrasound diagnosis apparatus 100 according to an embodiment.

Referring to FIG. 1, the ultrasound diagnosis apparatus 100 may include a probe 20, an ultrasound transceiver 100, an image processor 150, a display 160, a communication module 170, a memory 180, a user input unit 190, and a controller 195. Also, the above-mentioned components may be connected with each other via a bus 185, and the image processor 150 may include an image generator 155 , a cross-section information detector 130, and the display 160.

A person of ordinary skill in the art will understand that other general purpose components besides the components illustrated in FIG. 1 may be further included.

In some embodiments, the ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly, and according to embodiments, the ultrasound diagnosis apparatus 100 may include a plurality of probes 20.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 124 by delay times necessary for determining reception directionality, and the summing unit 128 generates ultrasound data by summing the echo signals processed by the reception delaying unit 126.

The image processor 150 generates an ultrasound image by scan-converting ultrasound data generated by the ultrasound transceiver 115.

The ultrasound image may be not only a grayscale ultrasound image obtained by scanning an object in an amplitude (A) mode, a brightness (B) mode, and a motion (M) mode, but also may represent motion of an object by using a Doppler image. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 141 extracts B mode components from ultrasound data and processes the B mode components. An image generator 155 may generate an ultrasound image indicating signal intensities as brightness based on the extracted B mode components 141.

Similarly, a Doppler processor 142 may extract Doppler components from ultrasound data, and the image generator 155 may generate a Doppler image indicating a movement of an object as colors or waveforms based on the extracted Doppler components.

The image generator 155 may generate a 2D ultrasound image or a 3D ultrasound image of an object, and may also generate an elastic image that shows a degree of deformation of an object 10 depending on pressure. Furthermore, the image generator 155 may display various pieces of additional information in an ultrasound image by using text and graphics. In addition, the generated ultrasound image may be stored in the memory 180.

The display 160 displays the generated ultrasound image. The display 160 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 160 according to embodiments.

The display 160 may include at least one of a liquid crystal display (LCD), a thin film transistor-liquid crystal display (TFT-LCD), an organic light-emitting diode (OLED) display, a flexible display, a 3D display, and an electrophoretic display.

Also, in the case where the display 160 and a user input unit configure a touchscreen by forming a layered structure, the display 160 may be used as not only an output unit but also an input unit that may receive information via a user's touch.

The touchscreen may be configured to detect even a touch pressure as well as a touch location and a touched area. Also, the touchscreen may be configured to detect not only a real-touch but also a proximity touch.

In the specification, a "real-touch" denotes a case where a pointer is actually touched onto a screen, and a "proximity-touch" denotes a case where a pointer does not actually touch a screen but is held apart from a screen by a predetermined distance. In the specification, a pointer denotes a touch tool for touching or proximity-touching a specific portion of a displayed screen. For example, a pointer may be an electronic pen, a finger, etc.

Although not shown in the drawings, the ultrasound diagnosis apparatus 100 may include various sensors inside or in the vicinity of a touchscreen in order to detect a direct touch or a proximity touch with respect to the touchscreen. An example of a sensor for detecting a touch with respect to the touchscreen includes a tactile sensor.

The tactile sensor denotes a sensor for detecting a contact of a specific object to a degree felt by a person or more. The tactile sensor may detect various information such as roughness of a contact surface, hardness of a contact object, and the temperature of a contact point.

Also, an example of a sensor for detecting a touch with respect to the touchscreen includes a proximity sensor. The proximity sensor denotes a sensor for detecting an object approaching a predetermined detection surface or the existence of an object in the neighborhood by using an electromagnetic force or an infrared ray without any mechanical contact.

An example of a proximity sensor includes a transmissive photoelectric sensor, a direct reflective photoelectric sensor, a mirror reflective photoelectric sensor, a high frequency oscillation type proximity sensor, a capacitive proximity sensor, a magnetic proximity sensor, an infrared proximity sensor, etc.

The communication module 170 is connected to a network 30 by wire or wirelessly to communicate with an external device or a server. The communication module 170 may exchange data with a hospital server or another medical apparatus in a hospital, which is connected thereto via a PACS. Furthermore, the communication module 170 may perform data communication according to the digital imaging and communications in medicine (DICOM) standard.

The communication module 170 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 170 may receive information about a diagnosis history or medical treatment schedule of a patient from a server and utilizes the received information to diagnose the patient. Furthermore, the communication module 170 may perform data communication not only with a server or a medical apparatus in a hospital, but also with a portable terminal of a medical doctor or patient.

The communication module 170 is connected to the network 30 by wire or wirelessly to exchange data with a server 32, a medical apparatus 34, or a portable terminal 36. The communication module 170 may include one or more components for communication with external devices. For example, the communication module 170 may include a local area communication module 171, a wired communication module 172, and a mobile communication module 173.

The local area communication module 171 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 172 refers to a module for communication using electric signals or optical signals. Examples of wired communication techniques according to an embodiment may include communication via a twisted pair cable, a coaxial cable, an optical fiber cable, and an Ethernet cable.

The mobile communication module 173 transmits or receives wireless signals to or from at least one selected from a base station, an external terminal, and a server on a mobile communication network. The wireless signals may be voice call signals, video call signals, or various types of data for transmission and reception of text/multimedia messages.

The memory 180 stores various data processed by the ultrasound diagnosis apparatus 100. For example, the memory 180 may store medical data related to diagnosis of an object, such as ultrasound data and an ultrasound image that are input or output, and may also store algorithms or programs which are to be executed in the ultrasound diagnosis apparatus 100.

The memory 180 may be any of various storage media, e.g., a flash memory, a hard disk drive, EEPROM, etc.

Furthermore, the ultrasound diagnosis apparatus 100 may utilize web storage or a cloud server that performs the storage function of the memory 180 online.

The user input unit 190 generates input data which a user inputs in order to control an operation of the ultrasound diagnosis apparatus 100. The user input unit 190 may include a hardware configuration such as a keypad, a mouse, a touchpad, a track ball, and a jog switch, but is not limited thereto, and may further include various configurations such as an electrocardiogram measurement module, a breathing measurement module, a voice recognition sensor, a gesture recognition sensor, a fingerprint recognition sensor, an iris recognition sensor, a depth sensor, and a distance sensor.

Particularly, the user input unit 190 may also include a touchscreen in which a touchpad and the display 160 form a layered structure.

In this case, the ultrasound diagnosis apparatus 100 according to an embodiment may display an ultrasound image of a predetermined mode and a control panel for an ultrasound image on the touchscreen. Also, the ultrasound diagnosis apparatus 100 may detect a user's touch gesture for an ultrasound image via the touchscreen.

The ultrasound diagnosis apparatus 100 according to an embodiment may physically include some buttons frequently used by a user from among buttons included in a control panel of a general ultrasound apparatus, and provide the rest of the buttons in the form of a GUI via the touchscreen.

The controller 195 may control all operations of the ultrasound diagnosis apparatus 100. In other words, the controller 195 may control operations among the probe 20, the ultrasound transceiver 100, the image processor 150, the communication module 170, the memory 180, and the user input unit 190 shown in FIG. 1.

All or some of the probe 20, the ultrasound transceiver 115, the image processor 150, the communication module 170, the memory 180, the user input unit 190, and the controller 195 may be implemented as software modules. However, embodiments of the present invention are not limited thereto, and some of the components stated above may be implemented as hardware modules. Furthermore, at least one selected from the ultrasound transceiver 115, the image processor 150, and the communication module 170 may be included in the controller 195. However, embodiments of the present invention are not limited thereto.

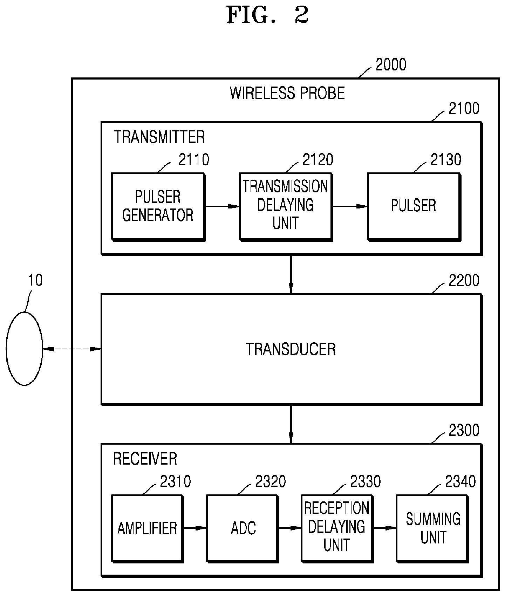

FIG. 2 is a block diagram showing a configuration of a wireless probe 2000 according to an embodiment. As described above with reference to FIG. 1, the wireless probe 2000 may include a plurality of transducers, and, according to embodiments, may include some or all of the components of the ultrasound transceiver 100 shown in FIG. 1.

The wireless probe 2000 according to the embodiment shown in FIG. 2 includes a transmitter 2100, a transducer 2200, and a receiver 2300. Since descriptions thereof are given above with reference to FIG. 1, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

The wireless probe 2000 may be a smart apparatus that may perform ultrasound scanning by including a transducer array. Specifically, the wireless probe 2000 is a smart apparatus, scans an object by using a transducer array, and obtains ultrasound data. Then, the wireless probe 2000 may generate and/or display an ultrasound image by using the obtained ultrasound data. The wireless probe 2000 may include a display, and display a screen including a user interface screen for controlling at least one ultrasound image and/or a scan operation of an object via the display.

While a user scans a patient's predetermined body portion, which is an object, by using the wireless probe 2000, the wireless probe 2000 and the ultrasound diagnosis apparatus 100 may continue to transmit/receive predetermined data via a wireless network. Specifically, while a user scans a patient's predetermined body portion, which is an object, by using the wireless probe 2000, the wireless probe 2000 may transmit ultrasound data to the ultrasound diagnosis apparatus 100 in real-time via the wireless network. The ultrasound data may be updated in real-time and transmitted from the wireless probe 2000 to the ultrasound diagnosis apparatus 100 as ultrasound scanning is performed continuously.

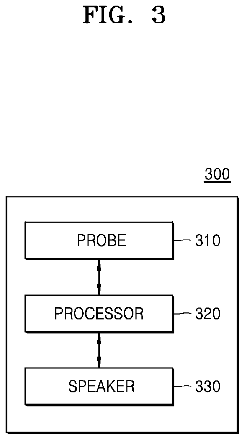

FIG. 3 is a block diagram of a configuration of an ultrasound diagnosis apparatus 300 according to an embodiment.

Referring to FIG. 3, the ultrasound diagnosis apparatus 300 according to the present embodiment may include a probe 310, a processor 320, and a speaker 330. However, all of the components shown in FIG. 3 are not essential components. The ultrasound diagnosis apparatus 300 may include more or fewer components than those shown in FIG. 3. Configurations and operations of the probe 310, the processor 320, and the speaker 330 will now be described in detail.

The probe 310 may include a plurality of transducers that convert ultrasound signals into electrical signals or vice versa. In other words, the probe 310 may include a transducer array consisting of a plurality of transducers. The plurality of transducers may be arranged in a one-dimensional (1D) or 2D array, and each of the plurality of transducers generates ultrasound signals separately or simultaneously. An ultrasound signal transmitted by each transducer is reflected off a discontinuous impedance surface within an object. Each transducer may convert a received reflected echo signal into an electrical reception signal. The probe 310 may transmit ultrasound signals to a fetus and receive echo signals reflected from the fetus.

The processor 320 acquires Doppler data based on echo signals. The processor 320 may also determine a fetus's heart rate based on period information of the Doppler data. The processor 320 may modulate, based on the fetus's heart rate, a fetus's heartbeat Doppler sound acquired from the Doppler data into a heartbeat sound. In this case, the heartbeat sound may be similar to a sound of a fetus's actual heartbeat. A heartbeat sound may be generated based on a heart rate. For example, as a heart rate increases, a period of a heartbeat sound may shorten. On the other hand, as the heart rate decreases, the period of heartbeat sound may increase.

The processor 320 may acquire gestational age information of a fetus based on Doppler data. The Doppler data may include Doppler-related images, graphs, values of specific parameters, etc. It will be understood by those of ordinary skill in the art to which the present embodiment pertains that the Doppler data may include other types of information. The gestational age information of the fetus may include at least one of a fetus's gestational age, and head circumference, abdominal circumference, femur length, abdominal thickness, body weight, transverse trunk diameter and crown rump length, all of which correspond to the fetus's gestational age, and embodiments are not limited thereto.

The processor 320 may modulate a heartbeat Doppler sound into a heartbeat sound according to gestational age information of a fetus and a fetus's heart rate.

The processor 320 may compare the fetus's heart rate with a standard heart rate of a plurality of fetuses corresponding to the gestational age information of the fetus. The standard heart rate for the plurality of fetuses is a representative value of heart rates of the plurality of fetuses. For example, the standard heart rate for the plurality of fetuses corresponding to the gestational age information of the fetus may be calculated by using a statistical method based on heart rate data with respect to the plurality of fetuses having the same gestational age as the fetus. In detail, the statistical method may include calculating an average of heart rates of a plurality of fetuses having the same gestational age as the fetus. Furthermore, the statistical method may include calculating an average of heart rates of fetuses that are in the same body weight group as the fetus, among a plurality of fetuses having the same gestational age as the fetus. In this case, the fetuses in the same body weight group have measured body weights that fall within a particular range around the fetus's body weight.

As another example, the processor 320 may compare the fetus's heart rate with a standard heart rate for fetuses that are in the same head and/or abdominal circumference group as the fetus, from among a plurality of fetuses having the same gestational age as the fetus. In other words, the processor 320 may compare the fetus's heart rate with a standard heart rate for fetuses having value(s) of specific parameter(s) that is (are) in the same group as that (those) of the fetus, from among a plurality of fetuses having the same gestational age as the fetus. A parameter may have different values according to a gestational age of the fetus and is used as an indicator for estimating fetal growth.

The processor 320 may determine modulation information based on a comparison result and a standard heartbeat sound for the plurality of fetuses. The processor 320 may modulate a heartbeat Doppler sound into a heartbeat sound by using the determined modulation information.

The standard heartbeat sound for the plurality of fetuses may be determined based on the standard heart rate for the plurality of fetuses corresponding to the gestational age information of the fetus.

In detail, if the fetus's heart rate is the same as the standard heart rate for the plurality of fetuses corresponding to the gestational age information of the fetus, the processor 320 may generate a fetus's heartbeat sound that is the same as the standard heartbeat sound for the plurality of fetuses. In this case, values that are within a particular range around a certain value may be considered to be "the same as" the certain value.

Otherwise, if the fetus's heart rate is different from the standard heart rate for the plurality of fetuses corresponding to the gestational age information of the fetus, the processor 320 may generate a heartbeat sound by correcting the standard heartbeat sound based on a difference between the fetus's heart rate and the standard heart rate.

The speaker 330 may output a heartbeat sound. Furthermore, when an ultrasound image in which a focus is on a fetus' s heart is displayed on the ultrasound diagnosis apparatus 300, the processor 320 may control the speaker 330 to output a heartbeat sound. The processor 320 may also control the speaker 330 to output a heartbeat Doppler sound acquired from Doppler data.

The processor 320 may control the speaker 330 to output prenatal music based on a state of the fetus's heart rate. In detail, if the fetus's heart rate is higher than a previously measured heart rate by a specific value, the processor 320 may control the speaker 330 to output calming prenatal music. Otherwise if the fetus's heart rate is lower than the previously measured heart rate by a specific value, the processor 320 may control the speaker 330 to output cheerful prenatal music.

The ultrasound diagnosis apparatus 300 may include a central arithmetic processor that controls all operations of the probe 310, the processor 320, and the speaker 330. The central arithmetic processor may be implemented as an array of a plurality of logic gates or a combination of a general purpose microprocessor and a memory for storing a program that can be run on the general purpose microprocessor. Furthermore, it will be appreciated by those of ordinary skill in the art to which the present embodiment pertains that the central arithmetic processor may be formed by different types of hardware.

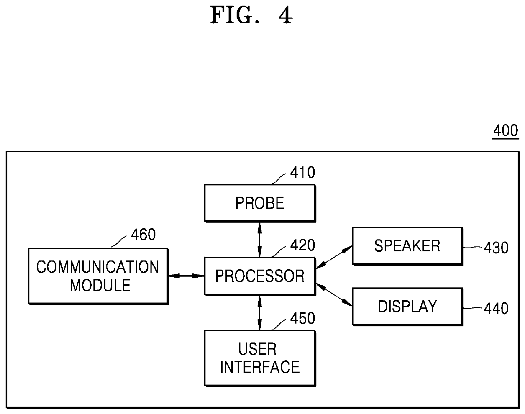

FIG. 4 is a block diagram of a configuration of an ultrasound diagnosis apparatus 400 according to another embodiment.

Referring to FIG. 4, the ultrasound diagnosis apparatus 400 according to the present embodiment may include a probe 410, a processor 420, a speaker 430, a display 440, a user interface 450, and a communication module 460.

Since the probe 410, the processor 420, and the speaker 430 included in the ultrasound diagnosis apparatus 400 of FIG. 1 respectively correspond to the probe 310, the processor 320, and the speaker 330 in the ultrasound diagnosis apparatus 300 described with reference to FIG. 3, descriptions already provided above with respect to FIG. 3 will be omitted below. The ultrasound diagnosis apparatus 400 may include more or fewer components than those shown in FIG. 4. Configurations and operations of the display 440, the user interface 450, and the communication module 460 will now be described in detail.

The display 440 displays a predetermined screen. In detail, the display 440 may display a predetermined screen according to control by the processor 420. The display 440 includes a display panel (not shown) and displays a medical image screen, etc. on the display panel.

The display 440 may display at least one of an ultrasound image, a Doppler spectrum, and heart rate information of a fetus.

Furthermore, the display 440 may display a waveform of a heartbeat sound. The display 440 may display the fetus's heart rate and the waveform of the heartbeat sound in such a manner that they are respectively distinguished from a standard heart rate of a plurality of fetuses corresponding to gestational age information of the fetus and a waveform of a standard heartbeat sound therefor.

The user interface 450 refers to a device via which data for controlling the ultrasound diagnosis apparatus 400 is received from a user. The processor 420 may control the display 440 to generate and output a screen of the user interface 450 for receiving a predetermined command or data from the user. The display 440 may display a screen for receiving an input of a heartbeat sound type on the display panel. In this case, heartbeat sound types may include at least one of a heartbeat sound type heard through a stethoscope, a preset resounding heartbeat sound type, and a preset majestic heartbeat sound type, and embodiments are not limited thereto.

The processor 420 may modulate a heartbeat Doppler sound into a heartbeat sound according to a determined heartbeat sound type. In detail, when the user requests generation of a heartbeat sound corresponding to a first sound type via the user interface 450, the processor 420 may modulate a heartbeat Doppler sound into the heartbeat sound corresponding to the first sound type, based on a standard heartbeat sound corresponding to a fetus's gestational age and its heart rate. In this case, the fetus's gestational age and heart rate may be acquired directly from fetal Doppler data within the ultrasound diagnosis apparatus 400 or be received from the user via the user interface 450.

The communication module 460 may receive and/or transmit data from and/or to an external device. For example, the communication module 460 may transmit a heartbeat sound to an external terminal. Furthermore, the communication module 460 may transmit fetal ultrasound images and Doppler data to the external terminal. For example, the external terminal may be a fetal-maternal terminal. The fetal-maternal terminal may receive a fetus's heartbeat sound file from the communication module 460. As another example, the external terminal may be a relay server of an application for providing fetal information to pregnant women or a server that manages medical records of the pregnant women. The communication module 460 may connect to a wireless probe or an external device via a communication network based on Wi-Fi or Wi-Fi Direct (WFD) technology. In detail, examples of a wireless communication network to which the communication module 460 can connect may include, but are not limited to, Wireless LAN (WLAN), Wi-Fi, Bluetooth, ZigBee, WFD, Ultra Wideband (UWB), Infrared Data Association (IrDA), Bluetooth Low Energy (BLE), and Near Field Communication (NFC).

The ultrasound diagnosis apparatus 400 may further include a storage device (not shown). The storage device may correspond to the memory 180 described with reference to FIG. 1. The storage device may store data related to an ultrasound image (e.g., an ultrasound image, ultrasound data, scan-related data, data related to diagnosis of a patient, etc.), data transmitted from an external device to the ultrasound diagnosis apparatus 400, etc. The data transmitted from the external device may include patient-related information, data necessary for diagnosis and treatment of a patient, a patient's past medical history, a medical work list corresponding to instructions regarding diagnosis of a patient, and the like. The storage device may store an ultrasound image, a Doppler spectrum, and heart rate information of a fetus. Furthermore, the storage device may store a plurality of standard heart rates and a plurality of standard heartbeat sounds respectively corresponding to weeks of gestation age. The storage device may also store results obtained by respectively comparing a fetus's heart rate and a waveform of a heartbeat sound with a standard heart rate and a waveform of a standard heartbeat sound for a plurality of fetuses corresponding to gestational age information of the fetus.

The ultrasound diagnosis apparatus 400 may include a central arithmetic processor that controls all operations of the probe 410, the processor 420, the speaker 430, the display 440, the user interface 450, the communication module 460, and the storage device. The central arithmetic processor may be implemented as an array of a plurality of logic gates or a combination of a general purpose microprocessor and a memory for storing a program that can be run on the general purpose microprocessor. Furthermore, it will be appreciated by those of ordinary skill in the art that the central arithmetic processor may be formed by different types of hardware.

Hereinafter, various operations performed by the ultrasound diagnosis apparatus 300 (400) and applications thereof will be described in detail. Although none of the probe 310 (410), the processor 320 (420), the speaker 330 (430), the display 440, the user interface 450, the communication module 460, and the storage device are specified, features and aspects that would be clearly understood by and are obvious to those of ordinary skill in the art may be considered as a typical implementation. The scope of the present inventive concept is not limited by a name of a particular component or a physical/logical structure.

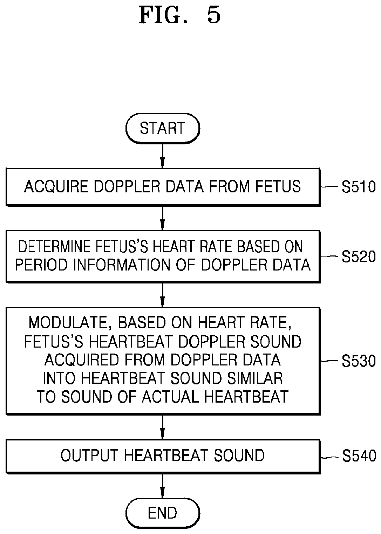

FIG. 5 is a flowchart of a method of operating an ultrasound diagnosis apparatus, according to an embodiment. Hereinafter, operations of the ultrasound diagnosis apparatus 300 may be applied in the same manner to the ultrasound diagnosis apparatus 400.

Referring to FIG. 5, the ultrasound diagnosis apparatus may acquire Doppler data from a fetus (S510). The ultrasound diagnosis apparatus may receive echo signals from the fetus and acquire Doppler data based on the received echo signals.

The ultrasound diagnosis apparatus may determine a fetus's heart rate based on period information of the Doppler data (S520). Furthermore, the ultrasound diagnosis apparatus may acquire gestational age information of the fetus based on the Doppler data. In this case, the gestational age information of the fetus may include at least one of a fetus's gestational age, and head circumference, abdominal circumference, femur length, abdominal thickness, transverse trunk diameter and crown rump length, all of which correspond to the fetus's gestational age, and embodiments are not limited thereto.

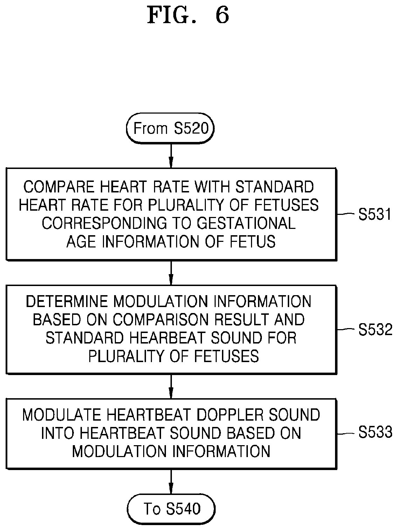

The ultrasound diagnosis apparatus may modulate, based on the fetus's heart rate, a fetus's heartbeat Doppler sound acquired from the Doppler data into a heartbeat sound that is similar to a sound of a fetus's actual heartbeat (S530). Furthermore, the ultrasound diagnosis apparatus may generate a heartbeat sound by taking into account the gestational age information of the fetus together with the fetus's heart rate. An operation, performed by the ultrasound diagnosis apparatus, of generating the heartbeat sound will be described in more detail below with reference to FIG. 6.

The ultrasound diagnosis apparatus may output the heartbeat sound (S540). When an ultrasound image in which a focus is on a fetus' s heart is displayed on a display of the ultrasound diagnosis apparatus, the ultrasound diagnosis apparatus may control a speaker of the ultrasound diagnosis apparatus to output the heartbeat sound.

FIG. 6 is a detailed flowchart of an operation of generating a heartbeat sound in a method of operating an ultrasound diagnosis apparatus, according to an embodiment.

Referring to FIG. 6, the ultrasound diagnosis apparatus may compare the fetus's heart rate with a standard heart rate of a plurality of fetuses corresponding to gestational age information of the fetus (S531).

In detail, the ultrasound diagnosis apparatus may compare the fetus's heart rate with a standard heart rate for fetuses having value(s) of specific parameter(s) that is (are) in the same group as that (those) of the fetus, from among a plurality of fetuses having the same gestational age as the fetus. A parameter may have different values according to a gestational age of the fetus and is used as an indicator for estimating fetal growth. For example, the specific parameter(s) may include at least one of a fetus's gestational age, and head circumference, abdominal circumference, femur length, abdominal thickness, body weight, transverse trunk diameter and crown rump length, all of which correspond to the fetus's gestational age. It will be understood by those of ordinary skill in the art that the specific parameter(s) may include a parameter other than the above-described parameters.

The ultrasound diagnosis apparatus may determine modulation information based on a result of comparison between the fetus's heart rate and the standard heart rate and a standard heartbeat sound for the plurality of fetuses (S532).

The ultrasound diagnosis apparatus may determine modulation information that is to be used for a heartbeat sound based on the standard heartbeat sound. In this case, the modulation information may include information about an amplitude, a frequency, and a phase of the standard heartbeat sound.

The ultrasound diagnosis apparatus may modulate a heartbeat Doppler sound into a heartbeat sound based on the determined modulation information (S533).

For example, if the fetus's heart rate is the same as the standard heart rate for the plurality of fetuses corresponding to the gestational age information of the fetus, the ultrasound diagnosis apparatus may acquire modulation information from the standard heartbeat sound for the plurality of fetuses. The ultrasound diagnosis apparatus may modulate, based on the modulation information, the heartbeat Doppler sound into a heartbeat sound that is the same as the standard heartbeat sound.

As another example, if the fetus's heart rate is different from the standard heart rate for the plurality of fetuses corresponding to the gestational age information of the fetus, the ultrasound diagnosis apparatus may acquire modulation information necessary for generating a heartbeat sound by correcting the standard heartbeat sound based on a difference between the fetus's heart rate and the standard heart rate. The ultrasound diagnosis apparatus may modulate, based on the modulation information, the heartbeat Doppler sound into a heartbeat sound that is the same as the standard heartbeat sound.

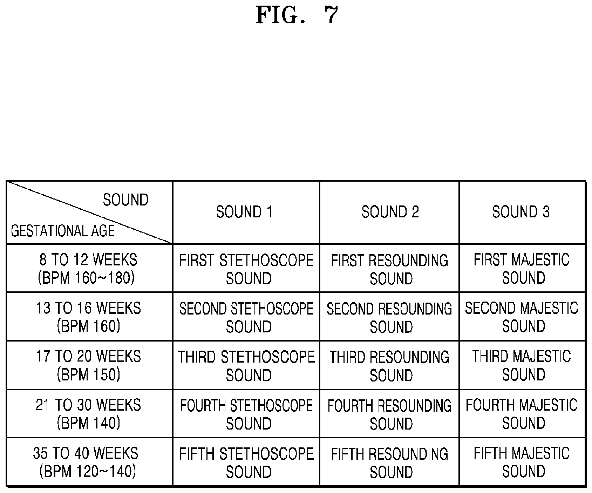

FIG. 7 is a table for explaining heartbeat sounds provided by an ultrasound diagnosis apparatus based on a gestational age and a heart rate of a fetus, according to an embodiment.

The ultrasound diagnosis apparatus may provide heartbeat sounds corresponding to a plurality of heartbeat sound types. As shown in FIG. 7, the plurality of heartbeat sound types may include a stethoscope sound type, a resounding sound type, and a majestic sound type. It will be understood by those of ordinary skill in the art that the plurality of heartbeat sound types may include other sound types.

As shown in FIG. 7, the ultrasound diagnosis apparatus may store heartbeat sounds corresponding to the plurality of heartbeat sound types for each gestational age group. In general, a fetal heart rate varies according to its gestational age. Referring to FIG. 7, a fetus's heart rate is in a range of between 160 and 180 beats per minute (bpm) at 8 to 12 weeks of gestational age, 160 bpm at 13 to 16 weeks, 140 bpm at 21 to 30 weeks, and in a range of between 120 and 140 bpm at 35 to 40 weeks. The above-described numerical values are merely an example for explaining the present embodiment, and it will be understood by those of ordinary skill in the art that the numerical values may vary according to embodiments.

According to an embodiment, the ultrasound diagnosis apparatus may receive echo signals from a fetus and acquire Doppler data from the echo signals. The ultrasound diagnosis apparatus may determine information about a fetus's heart rate and its gestational age from the Doppler data. For example, the ultrasound diagnosis apparatus may acquire information indicating that the fetus's gestational age is 19 weeks and its heart rate is 150 bpm. The ultrasound diagnosis apparatus may output a heartbeat sound according to a heartbeat sound type selected by the user. If the user selects a sound 1 type, the ultrasound diagnosis apparatus may output a third stethoscope sound.

In addition, while FIG. 7 shows that the gestational age of the fetus is divided into five gestational age groups: 8 to 12 weeks, 13 to 16 weeks, 17 to 20 weeks, 21 to 30 weeks, and 35 to 40 weeks, it may be divided into smaller gestational age groups. For example, the ultrasound diagnosis apparatus may store heartbeat sounds corresponding to each heartbeat sound type at two-week intervals

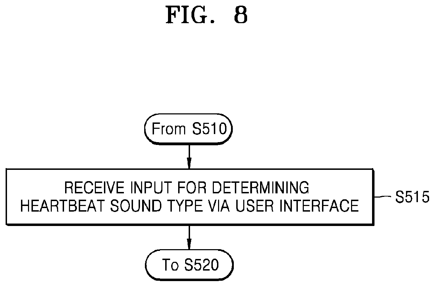

FIG. 8 is a flowchart of a method of operating an ultrasound diagnosis apparatus, according to another embodiment.

Referring to FIG. 8, the ultrasound diagnosis apparatus may receive an input for determining a heartbeat sound type via a user interface (S515). In this case, heartbeat sound types may include at least one of a heartbeat sound type heard through a stethoscope, a preset resounding heartbeat sound type, and a preset majestic heartbeat sound type.

The ultrasound diagnosis apparatus may modulate a heartbeat Doppler sound into a heartbeat sound according to a heartbeat sound type.

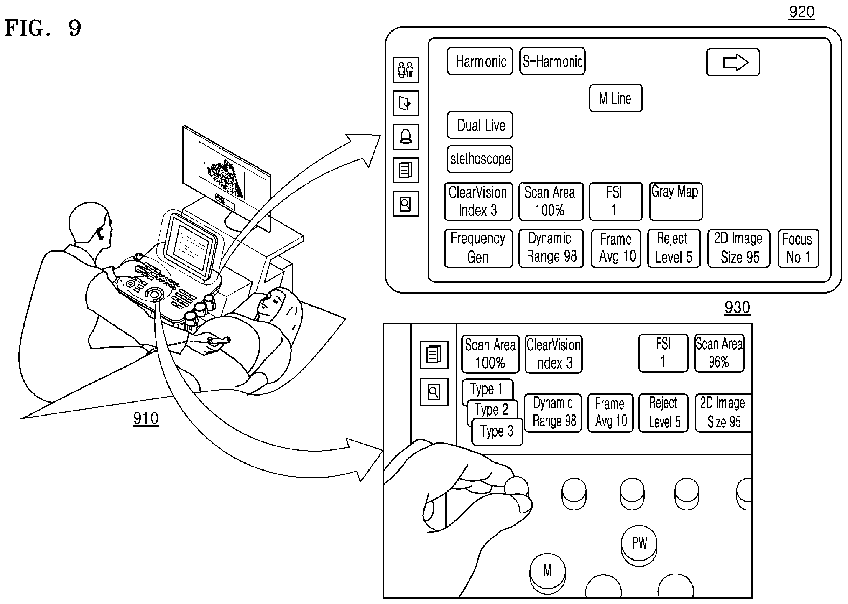

FIG. 9 illustrates a user interface provided in an ultrasound diagnosis apparatus, according to an embodiment.

As shown in 910 of FIG. 9, the ultrasound diagnosis apparatus may include the user interface. The user interface is a device via which data for controlling the ultrasound diagnosis apparatus 400 is received from a user. The user interface may include a display 920 configured to display a screen of the user interface for receiving a predetermined command or data from the user, separately from the display for displaying an ultrasound image. The user interface may further include hardware components 930, such as a keypad, a mouse, a touch panel, a touch screen, a track ball, and a jog switch, but is not limited thereto. Furthermore, the user interface may further include any of various other input tools including a voice recognition sensor, a gesture recognition sensor, an iris recognition sensor, a depth sensor, a distance sensor, etc.

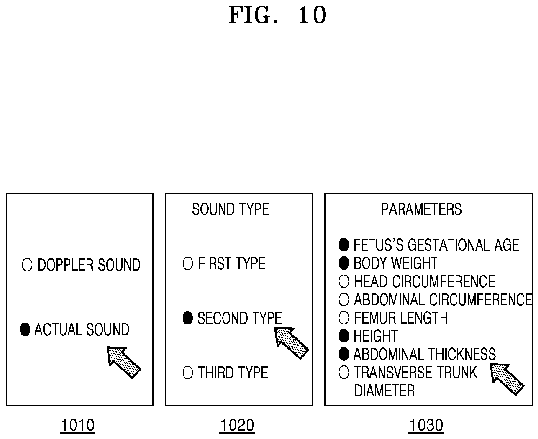

FIG. 10 illustrates a screen of a user interface provided in an ultrasound diagnosis apparatus, according to an embodiment.

As shown in 1010 of FIG. 10, the ultrasound diagnosis apparatus may provide a screen for determining whether a Doppler sound or actual sound is to be output as a fetus' heartbeat sound. The ultrasound diagnosis apparatus may output a heartbeat Doppler sound acquired from Doppler data or a heartbeat sound that is similar to a sound of a fetus's actual heartbeat. The ultrasound diagnosis apparatus may receive an input for selecting a heartbeat sound similar to an actual sound.

As shown in 1020 of FIG. 10, the ultrasound diagnosis apparatus may output fetus's heartbeat sounds respectively corresponding to various sound types. For example, the ultrasound diagnosis apparatus may output a fetus's heartbeat sound corresponding to a heartbeat sound type heard through a stethoscope, a preset resounding heartbeat sound type, or a preset majestic heartbeat sound type. The ultrasound diagnosis apparatus may receive an input for selecting one of a plurality of sound types.

As shown in 1030 of FIG. 10, the ultrasound diagnosis apparatus may generate the fetus's heartbeat sound based on at least one parameter related to the fetus. In this case, the at least one parameter may have different values according to a gestational age of the fetus and is used as an indicator for estimating fetal growth. Furthermore, the at least one parameter may include at least one of a fetus's gestational age, head circumference, abdominal circumference, femur length, abdominal thickness, body weight, transverse trunk diameter, and crown rump length. It will be understood by those of ordinary skill in the art that the at least one parameter may include another parameter.

As seen in 1030 of FIG. 10, the ultrasound diagnosis apparatus may receive an input for selecting specific parameters, i.e., a fetus's gestational age, body weight, height, and abdominal thickness.

If the fetus's heart rate is the same as the standard heart rate for the plurality of fetuses corresponding to a fetus's gestational age, body weight, height, and abdominal thickness, the ultrasound diagnosis apparatus may acquire modulation information from a standard heartbeat sound for the plurality of fetuses. The ultrasound diagnosis apparatus may modulate, based on the modulation information, a heartbeat Doppler sound into a heartbeat sound that is the same as the standard heartbeat sound.

In addition, as the number of parameters considered for generation of a fetus's heartbeat sound increases, the ultrasound diagnosis apparatus may output a more precise fetus's heartbeat sound.

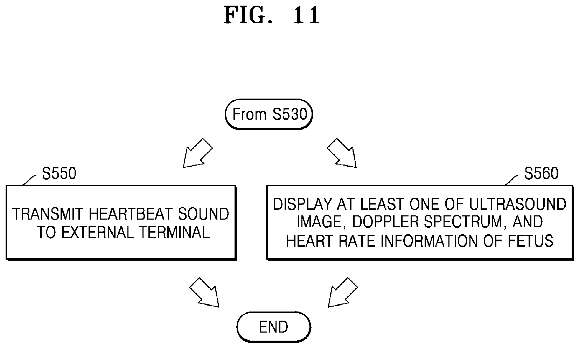

FIG. 11 is a flowchart of a method of operating an ultrasound diagnosis apparatus, according to another embodiment.

The ultrasound diagnosis apparatus may transmit the heartbeat sound to an external terminal (S550). The external terminal is a device for acquiring, storing, or using heartbeat sound data and/or data related to an ultrasound image and may be a medical imaging apparatus, a portable terminal, or any of all computing devices for using and processing medical images.

The ultrasound diagnosis apparatus may display at least one of an ultrasound image, a Doppler spectrum, and heart rate information of a fetus on a display of the ultrasound diagnosis apparatus (S560). In this case, the ultrasound diagnosis apparatus may process operation S560 in parallel with operation S550.

The ultrasound diagnosis apparatus may display a fetus's heart rate and a waveform of a heartbeat sound in such a manner that they are respectively distinguished from a standard heart rate of a plurality of fetuses corresponding to gestational age information of the fetus and a waveform of a standard heartbeat sound therefor.

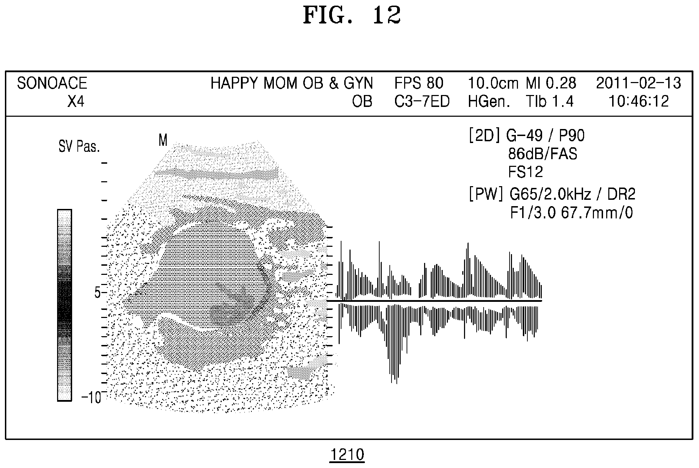

FIG. 12 illustrates a screen of a display on which an ultrasound diagnosis apparatus displays an ultrasound image, according to an embodiment.

The ultrasound diagnosis apparatus may receive echo signals from the fetus and acquire Doppler data based on the received echo signals. The ultrasound diagnosis apparatus may also obtain a Doppler spectrum and an ultrasound image based on the Doppler data. The ultrasound diagnosis apparatus may acquire a fetus's gestational age, and head circumference, abdominal circumference, femur length, abdominal thickness, body weight, transverse trunk diameter and crown rump length, all of which correspond to the fetus's gestational age. The ultrasound diagnosis apparatus may also display values of parameters acquired from the Doppler data on the display.

Referring to 1210 of FIG. 12, the ultrasound diagnosis apparatus may display an ultrasound image, a Doppler image, and a Doppler spectrum of a fetus on the display of the ultrasound diagnosis apparatus. In this case, the ultrasound image may include 3D and 2D ultrasound images. Furthermore, the Doppler image may be at least one of a color Doppler image, a power Doppler image, a tissue Doppler image, a vector Doppler image, and a spectral Doppler image, but is not limited thereto.

In addition, when an ultrasound image in which a focus is on a fetus' s heart is displayed on the display of the ultrasound diagnosis apparatus, the ultrasound diagnosis apparatus may control a speaker to output a fetus's heartbeat sound. The ultrasound diagnosis apparatus may output a heartbeat Doppler sound acquired from the Doppler data and/or a heartbeat sound that is similar to a sound of a fetus's actual heartbeat.

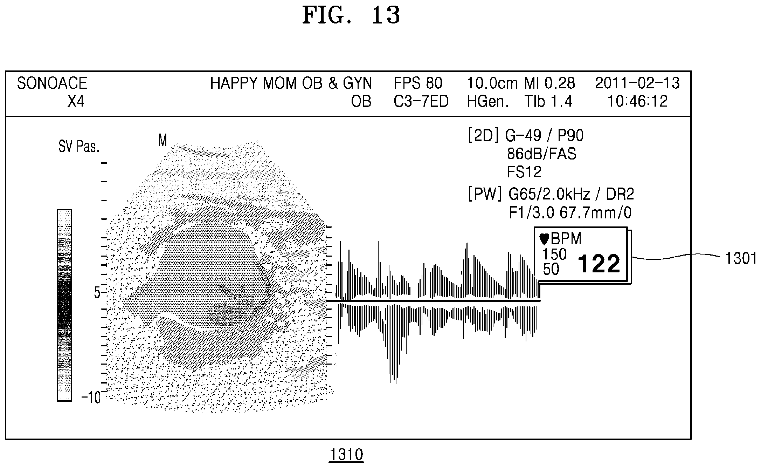

FIG. 13 illustrates a screen of a display on which an ultrasound diagnosis apparatus displays an ultrasound image, according to another embodiment.

As shown in 1310 of FIG. 13, the ultrasound diagnosis apparatus may display an ultrasound image, a Doppler spectrum, and heart rate information 1301 of the fetus on the display.

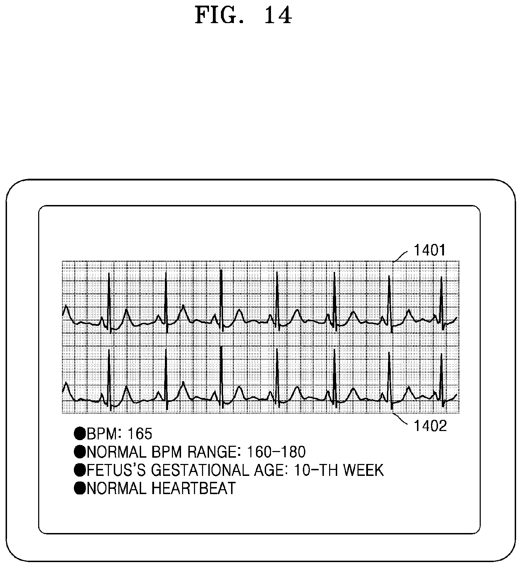

FIG. 14 illustrates a screen of a display on which an ultrasound diagnosis apparatus displays data related to an ultrasound image, according to an embodiment.

The ultrasound diagnosis apparatus may display a fetus's heart rate and a waveform of a heartbeat sound. In this case, the ultrasound diagnosis apparatus may display the fetus's heart rate and the waveform of the heartbeat sound so that they are respectively distinguished from a standard heart rate of a plurality of fetuses corresponding to gestational age information of the fetus and a waveform of a standard heartbeat sound therefor. Referring to FIG. 14, the ultrasound diagnosis apparatus may display, in separate regions, a waveform 1401 of a fetus's heartbeat sound and a waveform 1402 of a standard heartbeat sound, together with a comparison result. For example, the ultrasound diagnosis apparatus may display information indicating whether the waveform 1401 of the fetus's heartbeat sound is normal as a result of comparison with the waveform 1402. Furthermore, the ultrasound diagnosis apparatus may display information indicating that a heart rate corresponding to a fetus's gestational age is in a normal range.

Furthermore, the ultrasound diagnosis apparatus may display the waveform 1401 of the fetus's heartbeat sound and the waveform 1402 of the standard heartbeat sound in such a manner that they overlap each other in the same region. In this case, the ultrasound diagnosis apparatus may display the waveforms 1401 and 1402 as a solid line and a dashed line, respectively. Alternatively, the ultrasound diagnosis apparatus may display the waveforms 1401 and 1402 in different colors.

The above-described ultrasound diagnosis apparatus may be implemented by using a hardware component, a software component, and/or a combination of a hardware component and a software component. For example, the apparatus and the component described in the exemplary embodiments may be implemented by using one or more general-purpose computers or a special-purpose computers such as, for example, a processor, a controller, an arithmetic logic unit (ALU), a digital signal processor, a microcomputer, a field programmable array (FPA), a programmable logic unit (PLU), a microprocessor, or any device that may execute an instruction and respond thereto.

A processor may execute an operating system (OS) and one or more software applications executed on the OS. Also, the processor may access, store, manipulate, process, and generate data in response to execution of software.

For convenience of understanding, though description has been made to the case where one processor is used, a person of ordinary skill in the art will understand that the processor may include a plurality of processing elements and/or processing elements having a plurality of types. For example, the processor may include a plurality of processors, or one processor and one controller. Also, the processor may include a different processing configuration such as a parallel processor.

Software may include a computer program, a code, an instruction, or a combination of one or more of these, and configure the processor to operate as desired, or instruct the processor independently or collectively.

Software and/or data may be embodied permanently or temporarily in a certain type of a machine, a component, a physical device, virtual equipment, a computer storage medium or device, or a transmitted signal wave in order to allow the processor to analyze the software and/or data, or to provide an instruction or data to the processor. Software may be distributed on a computer system connected via a network, and stored and executed in a distributed fashion. Software and data may be stored in one or more non-transitory computer-readable recording media.

The methods according to exemplary embodiments may be embodied in the form of program commands executable through various computer means, which may be recorded on a non-transitory computer-readable recording medium. The non-transitory computer-readable recording medium may include program commands, data files, and data structures either alone or in combination. The program commands recorded on the non-transitory computer-readable recording medium may be those that are especially designed and configured for the inventive concept, or may be those that are known and available to computer programmers skilled in the art.

Examples of the non-transitory computer-readable recording medium include magnetic recording media such as hard disks, floppy disks, and magnetic tapes, optical recording media such as CD-ROMs and DVDs, magneto-optical recording media such as floptical disks, and hardware devices such as ROMs, RAMs, and flash memories that are especially configured to store and execute program commands.

Examples of the program commands include machine language codes that may be generated by a compiler, and high-level language codes that may be executed by a computer by using an interpreter.

The above hardware device may be configured to operate as one or more software modules in order to perform an operation of an exemplary embodiment, and vice versa.

Though the exemplary embodiments have been described by a limited number of exemplary embodiments and drawings, a person of ordinary skill in the art will make various modifications and changes from the above exemplary embodiments. For example, even when the described technologies are performed in an order different from the described method and/or components such as the described system, structure, apparatus, and circuit are coupled or combined in a form different from the described method, or replaced by other components or equivalents thereof, a proper result may be accomplished.

Therefore, the scope of the inventive concept should not be limited and determined by the described exemplary embodiments, but should be determined by not only the following claims but also equivalents thereof. It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the spirit and scope as defined by the following claims.

## Claims

1. An ultrasound diagnosis apparatus comprising:
a probe configured to receive echo signals from a fetus;
a processor configured to acquire Doppler data based on the received echo signals, determine a heart rate of the fetus based on period information of the Doppler data, and modulate, based on the determined heart rate, a heartbeat Doppler sound of the fetus, which is acquired from the Doppler data, into a heartbeat sound that is similar to a sound of an actual heartbeat; and
a speaker configured to output the heartbeat sound.

2. The ultrasound diagnosis apparatus of claim 1, wherein the processor is further configured to acquire gestational age information of the fetus based on the Doppler data and modulate the heartbeat Doppler sound into the heartbeat sound according to the gestational age information and the heart rate of the fetus.

3. The ultrasound diagnosis apparatus of claim 2, wherein the gestational age information of the fetus includes at least one of a gestational age of the fetus, and head circumference, abdominal circumference, femur length, abdominal thickness, transverse trunk diameter and crown rump length, all of which correspond to the gestational age of the fetus.

4. The ultrasound diagnosis apparatus of claim 1, wherein the processor is further configured to compare the heart rate with a standard heart rate of a plurality of fetuses corresponding to gestational age information of the fetus, determine modulation information based on a result of the comparing of the heart rate with the standard heart rate and a standard heartbeat sound for the plurality of fetuses, and modulate the heartbeat Doppler sound into the heartbeat sound based on the modulation information.

5. The ultrasound diagnosis apparatus of claim 1, further comprising a user interface configured to control an operation of the ultrasound diagnosis apparatus,
wherein the user interface is further configured to receive an input for determining a type of the heartbeat sound, and
wherein the processor is further configured to modulate the heartbeat Doppler sound into the heartbeat sound according to the type of the heartbeat sound.

6. The ultrasound diagnosis apparatus of claim 5, wherein the type of the heartbeat sound comprises at least one of a heartbeat sound heard through a stethoscope, a preset resounding heartbeat sound, and a preset majestic heartbeat sound.

7. The ultrasound diagnosis apparatus of claim 1, further comprising a display, wherein the processor is further configured to control the speaker to output the heartbeat sound when an ultrasound image in which a focus is on a fetus' s heart is displayed on the display.

8. The ultrasound diagnosis apparatus of claim 1, further comprising a communication module configured to transmit the heartbeat sound to an external terminal.

9. The ultrasound diagnosis apparatus of claim 1, further comprising a display configured to display at least one of an ultrasound image of the fetus, a Doppler spectrum of the fetus, and heart rate information thereof.

10. The ultrasound diagnosis apparatus of claim 9, wherein the display is further configured to display a waveform of the heartbeat sound.

11. The ultrasound diagnosis apparatus of claim 10, wherein the display is further configured to display the heart rate of the fetus and the waveform of the heartbeat sound to be respectively distinguished from a standard heart rate of a plurality of fetuses corresponding to gestational age information of the fetus and a waveform of a standard heartbeat sound for the plurality of fetuses.

12. The ultrasound diagnosis apparatus of claim 1, wherein the processor is further configured to control the speaker to output prenatal music based on a state of the heart rate of the fetus.

13. A method of operating an ultrasound diagnosis apparatus, the method comprising:
acquiring echo signals from a fetus and acquiring Doppler data based on the echo signals,
determining a heart rate of the fetus based on period information of the Doppler data;
modulating, based on the determined heart rate, a heartbeat Doppler sound of the fetus, which is acquired from the Doppler data, into a heartbeat sound that is similar to a sound of an actual heartbeat; and
outputting the heartbeat sound.

14. The method of claim 13, further comprising acquiring gestational age information of the fetus based on the Doppler data,
wherein the modulating of the heartbeat Doppler sound into the heartbeat sound comprises modulating the heartbeat Doppler sound into the heartbeat sound according to the gestational age information and the heart rate of the fetus.

15. The method of claim 13, wherein the modulating of the heartbeat Doppler sound into the heartbeat sound comprises:
comparing the heart rate with a standard heart rate of a plurality of fetuses corresponding to gestational age information of the fetus;
determining modulation information based on a result of the comparing of the
heart rate with the standard heart rate and a standard heartbeat sound for the plurality of fetuses; and
modulating the heartbeat Doppler sound into the heartbeat sound based on the modulation information.
